# EUROPÄISCHE PATENTSCHRIFT

(11) **EP 1 205 153 B1**
(45) Veröffentlichungstag und Bekanntmachung des Hinweises auf die Patenterteilung: **30.06.2004**
(21) Anmeldenummer: 01125553.6
(22) Anmeldetag: 25.10.2001
(51) Int. Cl.: A61B 17/70

(54) **Knochenschraube**
Bone screw
Vis d'ancrage osseux

(30) Priorität: 10.11.2000 DE 10055888; 27.12.2000 DE 10065397
(43) Veröffentlichungstag der Anmeldung: 15.05.2002
(73) Patentinhaber: BIEDERMANN MOTECH GmbH, 78054 Villingen-Schwenningen (DE)
(72) Erfinder: Harms, Jürgen, 76227 Karlsruhe (DE); Biedermann, Lutz, 78048 VS-Villingen (DE)
(74) Vertreter: Prüfer, Lutz H., Dipl.-Phys.

(56) Entgegenhaltungen:
- WO-A-98/34554
- US-A- 5 057 111
- US-A- 5 672 176
- US-A- 5 891 145
- US-A- 5 954 725
- US-A- 6 139 550

## Beschreibung

Die Erfindung betrifft eine Knochenschraube mit einem Gewindeabschnitt und einem Kopf und einem kopfseitigen Aufnahmeteil für die Aufnahme eines mit der Knochenschraube zu verbindenden Stabes, wobei das Aufnahmeteil eine offene erste Bohrung und einen im wesentlichen U-förmigen Querschnitt mit zwei freien mit einem Gewinde versehenen Schenkeln aufweist, und einer zweiten Bohrung an dem der ersten Bohrung gegenüberliegenden Ende, deren Durchmesser größer ist als der des Gewindeabschnittes und kleiner als der des Kopfes, und die den Sitz für den Kopf bildet, und einer mit dem Gewinde zusammenwirkenden Mutter bzw. Schraube.

Eine solche Knochenschraube ist beispielsweise aus der US 5,672,176 bekannt. Bei der bekannten Knochenschraube ist der Kopf kugelsegmentförmig ausgebildet. Der an die zweite Bohrung angrenzende Grund der ersten Bohrung ist ebenfalls kugelsegmentförmig ausgebildet, so daß der Kugelkopf auf diesem sphärischen Abschnitt aufliegt. Die durch den begrenzenden Rand gehende Ebene ist senkrecht zur Achse der ersten Bohrung ausgerichtet, und der Mittelpunkt der zweiten Bohrung fällt mit der Achse der ersten Bohrung zusammen. Dadurch wird erreicht, daß der den Kopf aufweisende Gewindeabschnitt in einem vorgegebenen Winkel von in der Regel bis zu 25° um die Achse der ersten Bohrung schwenkbar ist, so daß auch nach Einschrauben des Gewindeabschnittes in einen Wirbelkörper eine Ausrichtung des einen Stab aufnehmenden Aufnahmeteiles möglich ist. Dabei ist die Größe des Schwenkwinkels insofern begrenzt, als die zweite Bohrung in Abhängigkeit vom Durchmesser des Kopfes eine bestimmte Größe nicht überschreiten darf, damit der Kopf noch einen ausreichenden Halt in dem Aufnahmeteil besitzt.

Problematisch ist die Anwendung derartiger Knochenschrauben im Bereich der Halswirbel. Hier ist es erforderlich, daß wegen der kleinen Abmessungen der Halswirbel die Schrauben immer zu einer Seite und nach oben hin geschwenkt sein müssen, wobei eine größere Schwenkung als bei den größeren Brustwirbeln und Lendenwirbeln erforderlich ist.

Aus der US 5,891,145 ist eine Knochenschraube mit einem Schraubenelement, das einen Gewindeabschnitt und einen Kopf aufweist, und die ferner ein kopfseitiges Aufnahmeteil für die Aufnahme eines mit der Knochenschraube zu verbindenden Stabes aufweist, bekannt. Das Aufnahmeteil hat eine offene erste Bohrung und einen im wesentlichen U-förmigen Querschnitt mit zwei freien mit einem Gewinde versehenen Schenkeln und eine zweite Bohrung an dem der ersten Bohrung gegenüberliegenden Ende und einen Sitz für den Kopf. Ferner weist die Knochenschraube eine mit dem Gewinde der Schenkel zusammenwirkende Innenschraube auf. Der Sitz für den Kopf wird durch ein Keilelement bewirkt, welches seinerseits in einem in dem Aufnahmeteil vorgesehenenen weiteren Keilelement vorgesehen ist. Der Kopf der Knochenschraube hat gemäß einem Ausführungsbeispiel einen ringförmigen Wulst, der in einer Nut an der Innenseite des Keilelements sitzt oder in einem anderen Ausführungsbeispiel eine kegelige Außenfläche, die mit einer kegeligen Innenfläche des Keilelements zusammenwirkt. Ein kontinuierlicher Bereich von dreidimensionalen Winkelorientierungen der Knochenschraube in Bezug auf die Symmetrieachse des Aufnahmeteils läßt sich durch Verdrehen der Keilelemente gegeneinander erreichen. Ein Schwenken des Kopfes der Schraube in dem Aufnahmeteil ist jedoch bei dieser Knochenschraube nicht möglich.

Aufgabe der Erfindung ist es, eine Knochenschraube zu schaffen, die einen vergrößerten Schwenkwinkel zuläßt.

Diese Aufgabe wird durch eine in Patentanspruch 1 gekennzeichnete Knochenschraube gelöst.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine Seitenansicht einer ersten Ausführungsform, teilweise in geschnittener Darstellung;
- Fig. 2: einen vergrößerten Ausschnitt aus Fig. 1;
- Fig. 3: einen Seitenansicht, teilweise in geschnittener Darstellung einer zweiten Ausführungsform; und
- Fig. 4: eine entsprechende Darstellung einer weiteren Ausführungsform.

Die Knochenschraube weist das eigentliche Schraubenelement 1 mit einem Gewindeabschnitt 2 und einem Kopf 3 auf. Der Kopf ist angrenzend an den Gewindeabschnitt kugelsegmentförmig ausgebildet. Koaxial zur Gewindeachse und auf dem dem Gewindeabschnitt 2 gegenüberliegenden Ende weist der Kopf eine Ausnehmung 4 zum Ineingriffbringen mit einem Imbusschlüssel auf.

Die Knochenschraube umfaßt ferner ein zylindrisch ausgebildetes Aufnahmeteil 5. Dieses weist an seinem einen Ende eine axialsymmetrisch ausgebildete erste Bohrung 6 auf. Auf dem gegenüberliegenden Ende ist eine zweite Bohrung 7 vorgesehen, deren Durchmesser größer als der des Gewindeabschnittes 2 und kleiner als der des Kopfes 3 ist. Die erste Bohrung ist auf dem der zweiten Bohrung gegenüberliegenden Ende offen, und ihr Durchmesser ist so groß, daß das Schraubenelement 1 durch das offene Ende mit seinem Gewindeabschnitt 2 durch diese Bohrung hindurch und mit dem Kopf bis zum Grund der ersten Bohrung führbar ist. Der Grund der ersten Bohrung ist als ein zum offenen Ende hin sphärisch ausgebildeter Bereich ausgebildet, wobei der Radius im wesentlichen gleich dem Radius des kugelsegmentförmigen Abschnittes des Kopfes 3 ist. Ferner weist das Aufnahmeteil 5 eine zur Mitte des Teiles symmetrisch angeordnete U-förmige Ausnehmung 8 auf, deren Grund zu der zweiten Bohrung 7 hin gerichtet ist und deren beide Seitenschenkel 13, 14 sich zu dem der ersten Bohrung 6 hin gerichteten offenen Ende hin erstrecken. Am freien Ende der Schenkel 13, 14 ist ein Gewinde zum Ineingriffbringen mit einem als Mutter oder Schraube ausgebildeten Schraubelement vorgesehen. Die Mutter bzw. Schraube dient zum Fixieren eines in die U-förmige Ausnehmung 8 einzusetzenden Stabes, wobei die Mutter bzw. Schraube direkt oder über ein Druckelement auf den Stab einwirken kann.

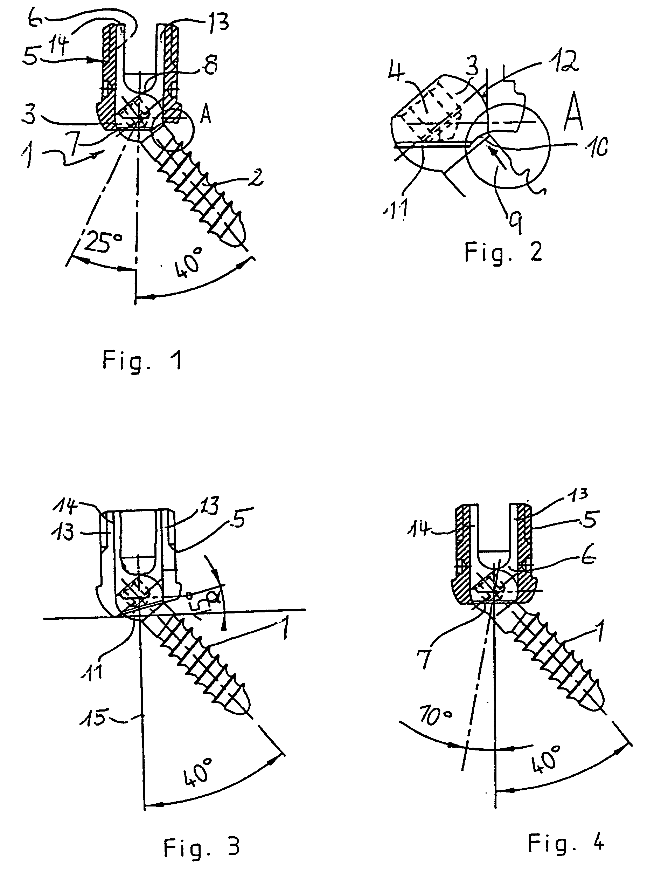

Bei der in den Fig. 1 und 2 gezeigten Ausführungsform ist in Richtung des Pfeiles 9, dessen Richtung in einer durch die Symmetrieachse der ersten Bohrung gehenden Ebene liegt und der um einen vorgegebenen Winkel gegen die Symmetrieachse geneigt ist, eine kreisförmige Ausfräsung 10 in dem Rand zwischen der Öffnungsebene 11 der zweiten Bohrung und dem Rand 12 der ersten Bohrung vorgenommen.

Auf diese Weise wird, wie aus den Figuren ersichtlich ist, erreicht, daß der Winkel zwischen der Achse des Schraubenelementes 1 und der Symmetrieachse der ersten Bohrung gegenüber dem sonst erreichbaren Winkel wesentlich vergrößert wird. Der Sitz des Schraubenelementes 1 in dem Aufnahmeteil bleibt dabei erhalten.

Bei der in Fig. 3 gezeigten zweiten Ausführungsform ist das Innere des Aufnahmeteiles 5 wie in der ersten Ausführungsform ausgebildet. Die Öffnungsebene 11, die die zweite Bohrung 7 begrenzt, ist bei dieser Ausführungsform um einen vorgegebenen Winkel α gegenüber der durch die zweite Bohrung 7 begrenzte Ebene geneigt, so daß das Lot dieser Ebene 11 mit der Symmetrieachse der ersten Bohrung 15 den Neigungswinkel einschließt. Im gezeigten Fall beträgt dieser Winkel α als Ausführungsbeispiel 15°. Auch bei dieser Version wird erreicht, daß das Schraubenelement 1 in der gezeigten Richtung um einen Winkel gegen die Symmetrieachse der ersten Bohrung schwenkbar ist, der wesentlich größer ist als der Winkel, der erreichbar ist in der üblichen Bauweise.

Sowohl bei der in Fig. 1 gezeigten Ausführungsform als auch bei der in Fig. 3 gezeigten Ausführungsform ist die Ausfräsung bzw. Abschrägung so gewählt, daß jeweils noch ein kleiner Randabschnitt verbleibt, der noch zu dem sphärischen Sitz gehört.

Bei einer nicht gezeigten vierten Ausführungsform ist der Mittelpunkt der zweiten Bohrung um ein geringes Maß, beispielsweise um 0,5mm, gegen die Symmetrieachse der ersten Bohrung seitlich versetzt ausgebildet. Diese seitliche Versetzung führt wiederum zu dem Ergebnis, daß der Kopf in der durch den sphärisch ausgebildeten Grund gebildeten Halterung gehalten, aber doch in einer Seitenrichtung eine größere Schwenkbreite erreicht wird.

Bei den oben beschriebenen Ausführungsbeispielen sind vier verschiedene Lösungsansätze dargestellt. Es ist auch möglich, die einzelnen Lösungsansätze miteinander zu kombinieren, also beispielsweise die Lösung nach dem ersten und zweiten Ausführungsbeispiel oder eine der beiden mit dem dritten und/oder vierten Ausführungsbeispiel oder gar alle vier Ausführungsbeispiele zu kombinieren, um so eine noch größere Schwenkungsmöglichkeit in wenigstens einer Richtung zu erzielen.

Bei den oben beschriebenen Ausführungsbeispielen ist der sphärische Grund der ersten Bohrung 6 jeweils als integraler Bestandteil des Aufnahmeteiles 5 ausgebildet. In einer abgewandelten Ausführungsform kann der sphärische Grund aber auch in einem entweder durch die erste Bohrung 6 einführbaren Fassungsteil oder in einem durch die zweite Bohrung 7 einführbaren Fassungsteil vorgesehen sein. Die Erfindung wird dann in sprechender Weise dahingehend angewendet, daß das Aufnahmeteil mit dem Einsatzstück als ein Element betrachtet wird und die oben beschriebenen Maßnahmen an diesem so zusammengesetzten Stück vorgenommen werden.

Die Knochenschraube bildenden Elemente sind vorzugsweise aus Titan gefertigt.

Bei der in Fig. 4 gezeigten Ausführungsform ist der das freie Ende der zweiten Bohrung begrenzende Rand zu Achse der ersten Bohrung gesehen symmetrisch ausgebildet. Die Asymmetrie wird dadurch erreicht, daß die Schraube 1 an ihrem an der Kugel bzw. dem Kugelsegment angreifenden Hals eine Ausnehmung bzw. Abfräsung 16 aufweist, so daß in der in Fig. 4 gezeigten Weise wie bei den vorher beschriebenen Ausführungsbeispielen der vergrößerte Schwenkwinkel erreicht werden kann.

## Patentansprüche

1. Knochenschraube mit einem einen Gewindeabschnitt (2) und einen kugelsegmentförmigen Kopf (3), der an dem dem Gewindeabschnitt (2) abgewandten Ende abgeflacht ist, aufweisenden Schraubenelement (1), einem kopfseitigen Aufnahmeteil (5) für die Aufnahme eines mit der Knochenschraube zu verbindenden Stabes mit einem Sitz für den Kopf (3), und einer mit dem Gewinde zusammenwirkenden Mutter bzw. Schraube, wobei das Aufnahmeteil (5) eine offene erste Bohrung (6) und einen im wesentlichen U-förmigen Querschnitt mit zwei freien, mit einem Gewinde versehenen Schenkeln aufweist,
**dadurch gekennzeichnet, daß** das Aufnahmteil (5) eine zweite Bohrung (7) an dem der ersten Bohrung (6) gegenüberliegenden Ende aufweist, deren Durchmesser größer ist als der des Gewindeabschnittes (2) und kleiner als der des Kopfes (3), und daß der das freie Ende der zweiten Bohrung (7) begrenzende Rand zur Achse der ersten Bohrung (6) gesehen unsymmetrisch ausgebildet ist.

2. Knochenschraube nach Anspruch 1, **dadurch gekennzeichnet, daß** der begrenzende Rand in einem Winkelbereich eine Ausfräsung (10) aufweist.

3. Knochenschraube nach Anspruch 1 oder 2, **dadurch gekennzeichnet, daß** das Lot der durch den begrenzenden Rand gehende Ebene gegen die Achse der ersten Bohrung (6) geneigt ist.

4. Knochenschraube nach einem der Ansprüche 1 bis 3, **dadurch gekennzeichnet, daß** der Mittelpunkt der von dem begrenzenden Rand umschlossenen Ebene gegen die Achse der ersten Bohrung (6) versetzt ist.

5. Knochenschraube mit einem einen Gewindeabschnitt (2) und einen kugelsegmentförmigen Kopf (3), der an dem dem Gewindeabschnitt abgewandten Ende abgeflacht ist, aufweisenden Schraubenelement (1), einem kopfseitigen Aufnahmeteil (5) für die Aufnahme eines mit der Knochenschraube zu verbindenden Stabes mit einem Sitz für den Kopf (3), und einer mit dem Gewinde zusammenwirkenden Mutter bzw. Schraube, wobei das Aufnahmeteil (5) eine offene erste Bohrung (6) und einen im wesentlichen U-förmigen Querschnitt mit zwei freien, mit einem Gewinde versehenen Schenkeln aufweist, **dadurch gekennzeichnet, daß** das Aufnahmteil (5) eine zweite Bohrung (7) an dem der ersten Bohrung (6) gegenüberliegenden Ende aufweist, deren Durchmesser größer ist als der des Gewindeabschnittes (2) und kleiner als der des Kopfes (3), und daß das Schraubenelement (1) an seinem an das Kugelsegment des Kopfes angrenzenden Hals eine Ausnehmung bzw. Fräsung (16) aufweist, wodurch eine Asymmetrie des Halses bezogen auf die Symmetrieachse der Schraube erreicht wird.

## Claims

1. Bone screw having a screw element (1) with a threaded portion (2) and a head (3) in the shape of a spherical segment which is flattened at the end remote from the threaded portion (2), with a receiving part (5) at the head end for receiving a rod to be connected to the bone screw, with a seat for the head (3), and with a nut or screw interacting with the thread, the receiving part (5) having an open first bore (6) and a substantially U-shaped cross section with two free arms provided with a thread, **characterized in that** the receiving part (5) has a second bore (7) at the end opposite to the first bore (6), the diameter of the second bore (7) being greater than that of the threaded portion (2) and smaller than that of the head (3), and **in that**, viewed relative to the axis of the first bore (6), the edge bounding the free end of the second bore (7) is of asymmetrical configuration.

2. Bone screw according to Claim 1, **characterized in that** the bounding edge has a countersink (10) in an angle region.

3. Bone screw according to Claim 1 or 2, **characterized in that** the perpendicular to the plane passing through the bounding edge is inclined relative to the axis of the first bore (6).

4. Bone screw according to one of Claims 1 to 3, **characterized in that** the mid-point of the plane enclosed by the bounding edge is offset relative to the axis of the first bore (6).

5. Bone screw having a screw element (1) with a threaded portion (2) and a head (3) in the shape of a spherical segment which is flattened at the end remote from the threaded portion, with a receiving part (5) at the head end for receiving a rod to be connected to the bone screw, with a seat for the head (3), and with a nut or screw interacting with the thread, the receiving part (5) having an open first bore (6) and a substantially U-shaped cross section with two free arms provided with a thread, **characterized in that** the receiving part (5) has a second bore (7) at the end opposite to the first bore (6), the diameter of the second bore (7) being greater than that of the threaded portion (2) and smaller than that of the head (3), and **in that**, at its neck adjoining the spherical segment of the head, the screw element (1) has a recess or sink (16), by which means an asymmetry of the neck relative to the axis of symmetry of the screw is obtained.

## Revendications

1. Vis à os avec un élément formant vis (1) présentant une partie filetée (2) et une tête (3) en forme de segment de sphère, qui est aplatie à l'extrémité opposée à la partie filetée (2), avec une partie de réception (5) du côté de la tête pour recevoir une tige qui doit être assemblée avec la vis à os et avec une assise pour la tête (3) et un écrou ou une vis coopérant avec le filetage, dans laquelle la partie de réception (5) présente un premier alésage (6) ouvert et une section sensiblement en forme de U avec deux bras libres pourvus d'un filetage, **caractérisée en ce que** la partie de réception (5) présente un deuxième alésage (7) à l'extrémité opposée au premier alésage (6), dont le diamètre est pl s grand que celui de la section filetée (2) et plus petit que celui de la tête (3), et **en ce que** le bord délimitant l'extrémité libre du deuxième alésage (7) est asymétrique par rapport à l'axe du premier alésage (6).

2. Vis à os selon la revendication 1, **caractérisée en ce que** le bord de délimitation présente une fraisure (10) dans une zone d'angle.

3. Vis à os selon la revendication 1 ou 2, **caractérisée en ce que** l'aplomb du plan passant par le bord de délimitation est incliné par rapport à l'axe du premier alésage (6).

4. Vis à os selon l'une des revendications 1 à 3, **caractérisée en ce que** le centre du plan délimité par le bord de délimitation est décalé par rapport à l'axe du premier alésage (6).

5. Vis à os avec un élément formant vis (1) présentant une partie filetée (2) et une tête (3) en forme de segment de sphère aplatie à l'extrémité opposée à la partie filetée, avec une partie de réception (5) du côté de la tête pour recevoir une tige à assembler avec la vis à os, avec une assise pour la tête (3) et un écrou ou une vis coopérant avec le filetage, dans laquelle la partie de réception (5) présente un premier alésage ouvert (6) et une section sensiblement en forme de U avec deux bras libres pourvus d'un filetage, **caractérisée en ce que** la partie de réception (5) présente un deuxième alésage (7) à l'extrémité opposée au premier alésage (6), dont le diamètre est plus grand que celui de la partie filetée (2) et plus petit que celui de la tête (3), et **en ce que** l'élément formant vis (1) présente sur son col contigu au segment de sphère de la tête un évidement ou une fraisure (16), ce qui permet d'obtenir une asymétrie du col par rapport à l'axe de symétrie de la vis.
